# EUROPEAN PATENT SPECIFICATION

(11) **EP 2 573 081 B1**
(45) Date of publication and mention of the grant of the patent: **12.07.2017**
(21) Application number: 11783277.4
(22) Date of filing: 18.05.2011
(51) Int. Cl.: A61K 31/496, A61P 1/16, A61P 43/00

(54) **PROPHYLACTIC AND/OR THERAPEUTIC AGENT FOR NON-ALCOHOLIC STEATOHEPATITIS**
PROPHYLAKTISCHES UND/ODER THERAPEUTISCHES MITTEL FÜR NICHT-ALKOHOLISCHE STEATOHEPATITIS
AGENT PROPHYLACTIQUE ET/OU THÉRAPEUTIQUE POUR UNE STÉATOHÉPATITE NON ALCOOLIQUE

(30) Priority: 19.05.2010 JP 2010115751
(43) Date of publication of application: 27.03.2013
(73) Proprietor: Kowa Company Ltd., Naka-ku Nagoya-shi Aichi 460-8625 (JP)
(72) Inventor: SHIBATA, Haruki, Higashimurayama-shi Tokyo 189-0022 (JP); YOSHINAKA, Yasunobu, Higashimurayama-shi Tokyo 189-0022 (JP); SHIBUYA, Kimiyuki, Higashimurayama-shi Tokyo 189-0022 (JP)
(74) Representative: Adam, Holger
(86) International application number: PCT/JP2011/002758
(87) International publication number: WO 2011/145340

(56) References cited:
- EP-A1- 1 666 038
- WO-A1-98/54153
- WO-A1-2005/020996
- WO-A1-2008/058383
- WO-A1-2008/091863
- WO-A1-2009/070781
- WO-A1-2010/042292
- MAMI IKENOYA ET AL: "A selective ACAT-1 inhibitor, K-604, suppresses fatty streak lesions in fat-fed hamsters without affecting plasma cholesterol levels", ATHEROSCLEROSIS, ELSEVIER IRELAND LTD, IE, vol. 191, no. 2, 1 April 2007 (2007-04-01) , pages 290-297, XP008158742, ISSN: 0021-9150, DOI: 10.1016/J.ATHEROSCLEROSIS.2006.05.048 [retrieved on 2006-07-03]
- DAIJIRO YASUDA ET AL.: 'Dislipidemia and steatohepatitis with visceral fat' JAPANESE JOURNAL OF CLINICAL MEDICINE vol. 67, no. 2, 01 February 2009, pages 344 - 349, XP008164342
- HIROMASA ISHII: 'NASH no Shikkan Gainen to Chiryo ni Tsuite' BIOMEDICINE & THERAPEUTICS vol. 43, no. 10, 2009, pages 109 - 114

## Description

### TECHNICAL FIELD

The present invention relates to a prophylactic and/or therapeutic agent for use in the treatment and/or prophylaxis of non-alcoholic steatohepatitis.

### BACKGROUND ART

Attention has been paid to metabolic syndrome as the concept of so-called lifestyle-related diseases such as type 2 diabetes mellitus. Metabolic syndrome is defined as a state having two or more of hyperglycemia, hypertension, and dyslipidemia in addition to visceral obesity. It progresses arteriosclerosis and increases the risk of development of myocardial infarction, cerebral infarction or the like.

Non-alcoholic fatty liver disease (hereinafter may be referred to as "NAFLD") is fatty hepatic disorder which is not due to drinking or clear pathogenesis (e.g., virus and autoimmunity). Recently, it is recognized as a phenotype in the liver of metabolic syndrome. Other various pathogenesis impairing fat or mitochondrial metabolism has been reported. NAFLD is further classified into simple steatosis which has a relatively benign prognosis and is caused only by fatty deposition in hepatocytes and nonalcoholic steatohepatitis
(hereinafter may be referred to as "NASH") which may result in fibrosis of hepatic tissues, liver cirrhosis, and hepatoma (Non-patent document 1).

As the developmental mechanism of NASH, the "two-hit theory" (Day et al.) is widely known (Non-patent document 2). That is, an imbalance between caloric intake and expenditure and storage of lipid in hepatocytes due to metabolic disorder based on insulin resistance are involved in the formation process of the fatty liver (1st hit). As the 2nd hit, an increase of oxidative stress due to energy metabolic load and an activation of the innate immune system accompanied thereby play an important role in the progression of fatty liver to NASH. Further, TNF-α, a typical inflammatory cytokine is known to worsen the condition of NASH. According to a report of study of the level of TNF-α expression in the hepatic tissues, as compared with obese patients (including simple fatty liver) except for those with NASH, the level of TNF-α expression in patients with NASH is significantly increased. Further, among the NASH patients, the level of TNF-α expression in those with liver fibrosis is significantly increased as compared with those without liver fibrosis. Thus, it has been reported that a strong correlation among TNF-α secreted by macrophages or activated T lymphocytes mainly infiltrated in hepatic tissues, necroinflammatory activity, and fibrosis is shown in the patients with NASH (Non-patent document 3).

Further, it has been reported that an abnormal increase in macrophages is observed in the liver in NASH model mice (Non-patent document 4).

The therapy of NASH is, in principle, an improvement in lifestyle based on the diet and exercise therapy for lifestyle-related diseases such as obesity, diabetes, dyslipidemia, and hypertension. However, it is actually difficult to improve the lifestyle and thus drug therapy targeting insulin resistance, oxidative stress, lipid metabolism abnormality, or hypertension which is considered to be an important factor for the progression of NASH is provided. As a therapeutic drug, an insulin resistance-improving drug such as thiazolidine derivatives (e.g., pioglitazone and rosiglitazone), which are ligands of nuclear receptor PPARγ associated with the potentiation of insulin sensitivity or a biguanide drug (e.g., metformin), i.e., an insulin resistance-improving drug is used. Further, an antioxidant such as vitamin E is used alone or in combination with vitamin C. Furthermore, as a therapeutic agent for lipid metabolism abnormality, fibrates (e.g., fenofibrate and bezafibrate) which is a PPARα agonist and a statin formulation, probucol, or the like is expected. As a therapeutic agent for hypertension, an angiotensin II type 1 receptor antagonist (ARB) is expected. Particularly, fibrates or statins are expected from the viewpoint of their anti-inflammatory activities. However, there are few reports on studies with a high evidence level, and a highly recommended method of treating NASH has not been established. The number of individuals affected with metabolic syndrome is increasing worldwide. Accordingly, the number of the patients with NASH is expected to increase, and it is desired to establish a method of treating NASH (Non-patent documents 1 and 5).

On the other hand, Patent document 1 describes that a compound represented by the following formula (1), a salt thereof, or a solvate of the same:

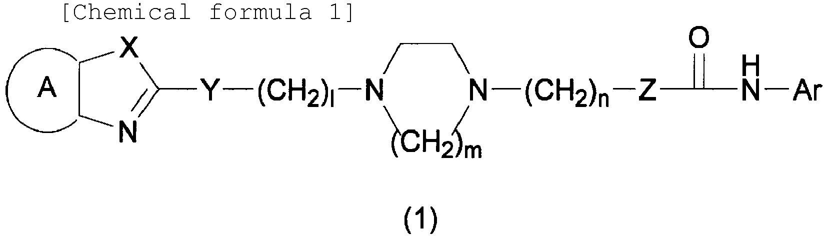

has an acyl coenzyme A: cholesterol acyltransferase (ACAT)-inhibiting activity, can be used as an ACAT inhibitor, intracellular cholesterol transport inhibitor, blood cholesterol-lowering agent, or macrophage foaming inhibitor, and is useful as a therapeutic agent for a disease such as hyperlipemia, arteriosclerosis, cervical and cerebral arteriosclerosis, cerebrovascular disorder, ischemic heart disease, coronary arteriosclerosis, nephrosclerosis, arteriosclerotic nephrosclerosis, arteriocapillary sclerosis nephrosclerosis, malignant nephrosclerosis, ischemic bowel disease, acute mesenteric vascular occlusion, chronic intestinal angina, ischemic colitis, aortic aneurysm, and arteriosclerosis obliterans (ASO). Further, Patent document 2 describes a
2-[4-[2-(benzimidazol-2-ylthio)ethyl]piperazin-1-yl]-N-[2,4 -bis(methylthio)-6-methyl-3-pyridyl]acetamide represented by the following formula (2), a salt thereof, or a solvate of the same:

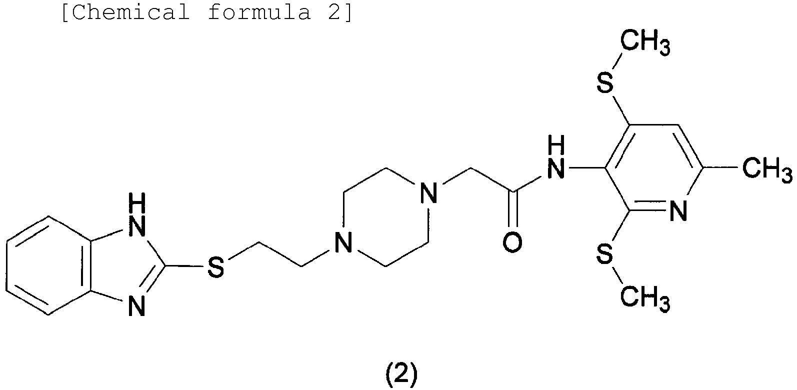

stabilizes an unstable and rupturable lipid-rich plaque which is a lesion of atherosclerosis, and is useful for prophylaxis of thrombus formation caused by the rupture of the lipid-rich plaque, prevention and/or treatment of acute coronary syndrome (ACS), prevention and/or treatment of acute myocardial infarction, prevention and/or treatment of unstable angitis, and prevention and/or treatment of peripheral arterial occlusive disease. However, the patent documents have neither description nor suggestion of the effects of these compounds on NASH.

### PRIOR ART LITERATURE

### PATENT DOCUMENT

Patent Document 1: WO 1998/054153
Patent Document 2: WO 2005/020996

### NON-PATENT DOCUMENT

Non-Patent Document 1: Nugent C et al., Nat. Clin. Pract. Gastroenterol. Hepatol., 8, 432-41 (2007)
Non-Patent Document 2: Day CP, et al, Gastroenterology, 114, 842-845 (1998)
Non-Patent Document 3: Crespo J, et al., Hepatology, 34, 1158-63 (2001)
Non-Patent Document 4: Kudo H, et al., Liver Int., 29, 988-996 (2009)
Non-Patent Document 5: Hirofumi Uto, et al., The Medical Frontline 97, 1683-87 (2008)

Atherosclerosis 191 (2007) 290-297 discloses a ACAT inhibitor, K-604, which suppresses fatty streak lesions in fat-fed hamsters without affecting plasma cholesterol levels.

WO 2008/058383 relates to the use of ACAT inhibitors for preventing or treating fibrosis for preventing or reducing collagen deposition in a tissue and in the prevention and reduction of excessive fibrous connective tissue.

WO 2010/042292 discloses a method for decreasing the size and density of amyloid plaques comprising administering to a subject in need of treatment an agent that selectively inhibits the activity of Acyl-CoA: Cholesterol Acyltranserfase 1 thereby decreasing the size and density of amyloid plaques in the subject.

EP 1 666 038 discloses a method of stabilizing lipid rich plaques and a method of preventing the rupture thereof characterized that an effective amount of
2-[4-[2-(benzimidazol-2-ylthio)ethyl]piperazin-1-yl]-N-[2,4 -bis(methylthio)-6-methyl-3-pyridyl]acetamide, its acid adduct salt or a hydrate thereof is administered to patients with lipid-rich plaques.

WO 2008/091863 discloses a method of treating non-alcoholic fatty liver disease, non-alcoholic steatohepatitis and other fibrotic diseases of the liver in a subject by modulating PPARδ with sulfonyl-substituted bicyclic compounds and compositions as pharmaceuticals.

### SUMMARY OF THE INVENTION

### PROBLEMS TO BE SOLVED BY THE INVENTION

The number of individuals affected with metabolic syndrome is increasing worldwide. Further, the number of patients with NASH is expected to increase in future. However, as for a therapy for NASH, there are few reports on studies with a high evidence level. Currently, a highly recommended method of treating NASH has not been established.

An objective of the present invention is to provide a novel prophylactic and/or therapeutic agent for non-alcoholic steatohepatitis (NASH) which is useful for prevention and treatment of NASH.

### MEANS FOR SOLVING THE PROBLEMS

In order to find a compound useful for prevention and treatment of non-alcoholic steatohepatitis (NASH), the present inventors have investigated the compound useful for prevention and treatment of NASH using LDL receptor-knockout mice, i.e., model animals with NASH. As a result, it has been unexpectedly found that
2-[4-[2-(benzimidazol-2-ylthio)ethyl]piperazin-1-yl]-N-[2,4 -bis(methylthio)-6-methyl-3-pyridyl]acetamide or a salt thereof does not affect the plasma lipid, has a direct effect on the liver, specifically reduces the levels of cholesterol and triglyceride in the liver, and inhibits the development of fatty liver and hepatic inflammation, i.e., characteristic conditions of NASH and thus have completed the present invention.

That is, the present invention provides 2-[4-[2-(benzimidazol-2-ylthio)ethyl]piperazin-1-yl]-N-[2,4 -bis(methylthio)-6-methyl-3-pyridyl]acetamide, an acid-addition salt thereof, or a solvate of the same for use in the treatment and/or prophylaxis of non-alcoholic steatohepatitis.

Further disclosed is a prophylactic and/or therapeutic method for non-alcoholic steatohepatitis in which an effective amount of
2-[4-[2-(benzimidazol-2-ylthio)ethyl]piperazin-l-yl]-N-[2,4 -bis(methylthio)-6-methyl-3-pyridyl]acetamide, an acid-addition salt thereof, or a solvate of the same is administered to needy patients.

Further disclosed is the use of
2-[4-[2-(benzimidazol-2-ylthio)ethyl]piperazin-l-yl]-N-[2,4 -bis(methylthio)-6-methyl-3-pyridyl]acetamide, an acid-addition salt thereof, or a solvate of the same to produce a prophylactic and/or therapeutic agent for non-alcoholic steatohepatitis.

### EFFECTS OF THE INVENTION

The present invention provides a novel prophylactic and/or therapeutic agent for use in the treatment and/or prophylaxis of non-alcoholic steatohepatitis (NASH).

There are few reports of a therapeutic agent for non-alcoholic steatohepatitis (NASH) with a high evidence level,
and a highly recommended method of treating NASH has not been established. The number of patients with NASH is expected to increase worldwide in future, and it is desired to establish a method of treating NASH. The present invention provides a novel prophylactic and/or therapeutic agent for use in the treatment and/or prophylaxis of non-alcoholic steatohepatitis (NASH) which does not affect the plasma lipid, has a direct effect on the liver, specifically reduces the levels of cholesterol and triglyceride in the liver, significantly inhibits TNF-α expression in the liver, and significantly reduces the level of macrophages in the liver.

### BRIEF DESCRIPTION OF THE DRAWINGS

FIG. 1 is a view showing total cholesterol (TC) in the plasma (FIG. 1A) and triglyceride (TG) in the plasma (FIG. 1B) when the compound A (3 mg/kg) of the present invention was administered in Example 1.
FIG. 2 is a view showing total cholesterol (TC) in the liver (FIG. 2A) and triglyceride (TG) in the liver (FIG. 2B) when the compound A (3 mg/kg) of the present invention was administered in Example 1.
FIG. 3 is a view showing the gene expression level of inflammatory M1 macrophage marker (CD11c) in the liver (FIG. 3A) and the gene expression level of inflammatory cytokine (TNF-α) in the liver (FIG. 3B) when the compound A (3 mg/kg) of the present invention was administered in Example 1.
FIG. 4 shows plasma ALT levels in a normal mouse group, a C57BL/6N-NASH mouse control group, and a group in which the compound A is administered to C57BL/6N-NASH mice.
FIG. 5 shows the TC content in the liver of a normal mouse group, a C57BL/6N-NASH mouse control group, and a group in which the compound A is administered to C57BL/6N-NASH mice.
FIG. 6 shows the TG content in the liver of a normal mouse group, a C57BL/6N-NASH mouse control group, and a group in which the compound A is administered to C57BL/6N-NASH mice.
FIG. 7 shows the photographic results of respective liver tissues (HE-stained) of a normal mouse group, a C57BL/6N-NASH mouse control group, and a group in which the compound A is administered to C57BL/6N-NASH mice. The original is a color picture. Arrows (→) in FIG. 7 show lipid droplets.
FIG. 8 shows plasma ALT levels in an MCD-fed KKA^{y} mouse control group and a group in which the compound A is administered to MCD-fed KKA^{y} mice.
FIG. 9 shows the TC content in the liver of an MCD-fed KKA^{y} mouse control group and a group in which the compound A is administered to MCD-fed KKA^{y} mice.
FIG. 10 shows the TG content in the liver of an MCD-fed KKA^{y} mouse control group and a group in which the compound A is administered to MCD-fed KKA^{y} mice.
FIG. 11 shows the photographic results of liver tissues (HE-stained) of an MCD-fed KKA^{y} mouse control group and a group in which the compound A is administered to MCD-fed KKAy mice. The original is a color picture.
FIG. 12 shows the photographic results of liver tissues (Azan-stained) of an MCD-fed KKA^{y} mouse control group and a group in which the compound A is administered to MCD-fed KKAy mice. The original is a color picture.

### MODES FOR CARRYING OUT THE INVENTION

2-[4-[2-(benzimidazol-2-ylthio)ethyl]piperazin-l-yl]-N-[2,4-bis(methylthio)-6-methyl-3-pyridyl]acetamide, an acid-addition salt thereof, or a solvate of the same to be used in the present invention can be produced by, for example, a method described in WO 1998/054153, WO 2005/020996 or WO 2004/076441. These can be formulated in accordance with the methods described in these references.

The acid which forms the acid-addition salt of the present invention is not particularly limited as long as it is a pharmaceutically acceptable acid. Examples thereof include an inorganic acid such as sulfuric acid, hydrochloric acid, nitric acid, phosphoric acid, and hydrobromic acid; and an organic acid such as acetic acid, lactic acid, succinic acid, tartaric acid, malic acid, maleic acid, citric acid, fumaric acid, methanesulfonic acid, and p-toluenesulfonic acid. The acid is preferably an inorganic acid, more preferably hydrochloric acid.

Examples of the solvate of the present invention include hydrates and alcoholates (e.g., ethanol solvates). Preferable examples thereof include hydrates.

2-[4-[2-(benzimidazol-2-ylthio)ethyl]piperazin-l-yl]-N-[2,4-bis(methylthio)-6-methyl-3-pyridyl]acetamide, an acid-addition salt thereof, or a solvate of the same in the present invention is preferably monohydrochloride of 2-[4-[2-(benzimidazol-2-ylthio)ethyl]piperazin-1-yl]-N-[2,4 -bis(methylthio)-6-methyl-3-pyridyl]acetamide.

As for non-alcoholic steatohepatitis (NASH), it is known that not only the levels of TC and TG in the liver is increased but also the expression level of inflammatory cytokine TNF-α in the liver is increased (See Non-patent document 3), and further the level of macrophages in the liver is increased (See Non-patent document 4).

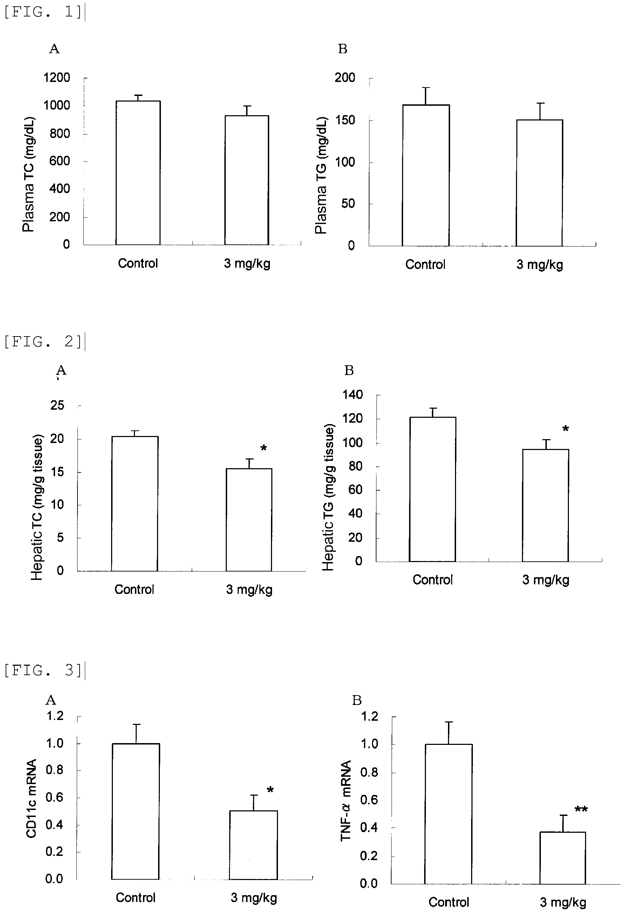

As described in the following Examples, when 2-[4-[2-(benzimidazol-2-ylthio)ethyl]piperazin-l-yl]-N-[2,4 -bis(methylthio)-6-methyl-3-pyridyl]acetamide, an acid-addition salt thereof, or a solvate of the same in the present invention was administered in a dose not significantly affecting the levels of TC and TG in the blood plasma to male LDL receptor-knockout mice, i.e., model animals with NASH, the levels of TC and TG in the liver were significantly decreased, and further the expression level of inflammatory cytokine TNF-α in the liver and the level of macrophage CD11c were significantly decreased. Particularly, the significant decrease in the levels of TC and TG in the liver in a dose not significantly affecting the levels of TC and TG in the blood plasma (See FIGS. 1 and 2) shows that the active ingredient of the present invention has a direct activity to the liver, which differs from the conventionally reported activity. It can be demonstrated that the effect is based on the activity which has been found out for the first time by the present invention.

Therefore,
2-[4-[2-(benzimidazol-2-ylthio)ethyl]piperazin-1-yl]-N-[2,4 -bis(methylthio)-6-methyl-3-pyridyl]acetamide, an acid-addition salt thereof, or a solvate of the same is effective as a prophylactic and/or therapeutic agent for non-alcoholic steatohepatitis in mammals including humans.

The present invention contains, as an active ingredient, 2-[4-[2-(benzimidazol-2-ylthio)ethyl]piperazin-1-yl]-N-[2,4 -bis(methylthio)-6-methyl-3-pyridyl]acetamide, an acid-addition salt thereof, or a solvate of the same. The active ingredient of the present invention may be useful not only as a prophylactic and/or therapeutic agent for non-alcoholic steatohepatitis in mammals including humans but also as a prophylactic and/or therapeutic agent for non-alcoholic fatty liver disease (NAFLD) in mammals including humans as shown in the following Examples.

As described in the following Examples, the active ingredient of the present invention is useful as a reducing agent for cholesterol in the liver, a reducing agent for triglyceride in the liver, an inhibiting agent for TNF-α expression in the liver, and a reducing agent for macrophage in the liver in mammals including humans.

The medicine of the present invention can be independently formulated into a dosage form such as a tablet, capsule, granule, powder, lotion, ointment, injection, or suppository. Alternatively, it can be formulated into such a form using a carrier such as a pharmaceutically acceptable solvent, excipient, binder, or diluent. These formulations can be produced by a known method. For example, when producing a formulation for oral administration, the drug can be produced by formulating with an appropriate combination of a solvent such as Tragacanth gum, Arabia gum, sucrose esters, lecithin, olive oil, soybean oil, and PEG400; an excipient such as starch, mannitol, and lactose; a binder such as
carboxymethylcellulose sodium and hydroxypropylcellulose; a disintegrator such as crystalline cellulose and carboxymethyl-cellulose calcium; a lubricant such as talc and magnesium stearate; a flow improver such as light anhydrous silicic acid; and a diluent such as corn starch.

The medicine of the present invention is orally or parenterally administered. The dosage of the medicine of the present invention varies depending on the weight, age, sex, and conditions of patients. In the case of normal adults, the dosage amount of
2-[4-[2-(benzimidazol-2-ylthio)ethyl]piperazin-1-yl]-N-[2,4 -bis(methylthio)-6-methyl-3-pyridyl]acetamide is from 0.01 to 1000 mg per day, preferably from 0.1 to 200 mg per day, in 1 to 3 divided doses.

### EXAMPLES

Hereinafter, the present invention will be more specifically described with reference to the Examples. However, the present invention is not limited to these Examples.

### Example 1 Effect in LDL receptor-knockout mouse NASH model

An effect in LDL receptor-knockout mice fed with the Western diet which were known to develop fatty liver and hepatic inflammation, i.e., characteristic conditions of NASH (Yoshimatsu M. et al. Int J Exp Pathol., 85(6), 335-43 (2004)) was examined. In the present test, monohydrochloride of 2-[4-[2-(benzimidazol-2-ylthio)ethyl]piperazin-1-yl]-N-[2,4 -bis(methylthio)-6-methyl-3-pyridyl]acetamide (hereinafter referred to as "compound A") was used.

1) Used animal:
   Male LDL receptor-knockout mice
   (B6.129S7-Ldlr<tm1Her>/J, Jackson Laboratories) were used for the experiment. The mice aged around 8 weeks were allowed to freely intake Teklad Custom Research Diet (TD.88137, manufactured by Harlan Teklad) as a Western diet for 12 weeks to cause them to develop NASH.
2) Group constitution:
   The mice were separated into a control group (hydrochloride added purified water) and a compound A (3 mg/kg, as free form) administered group so that there is no difference in the weights between the groups.
3) Administration of drug:
   The dose was calculated by 10 mL/kg weight.
      Hydrochloride added purified water and each drug solution were orally administered to the control group and the compound A administered group, respectively, at an interval of at least 6 hours, once in the morning and once in the afternoon. The mice aged around 8 weeks were fed with the Western diet, and the drug solution was administered at the same time. The dosing period was 12 weeks.
4) Observation and examination methods:
   After the end of administration, the mice were fasted overnight. The blood was collected from the abdominal portion of vena cava under anesthesia with pentobarbital sodium (50 mg/kg) and the plasma was fractionated. After collecting the blood, the liver was extracted and frozen for preservation. A part of the liver was frozen in ISOGEN (NIPPON GENE, CO., LTD.) and preserved to analyze the gene expression.

Total cholesterol (TC) and triglyceride (TG) in the plasma were measured using Cholesterol E Test Wako and Triglyceride E Test Wako (both manufactured by Wako Pure Chemical Industries, Ltd.), respectively. Measurement of liver lipid was performed in the following manner. Chloroform and methanol (at a ratio of 2:1) were added to the frozen and preserved liver, which was homogenized, and the resultant mixture was allowed to stand at 4°C longer than overnight, followed by filtration. Apart of the filtrate was fractionated, followed by evaporation and drying. The resultant product was dissolved in isopropanol. TC and TG concentrations were measured using Cholesterol E-Test Wako and Triglyceride E-Test Wako, respectively.

Liver gene expression analysis was performed by extracting RNA from the liver and measuring expression levels of TNF-α genes and CD11c genes in adhesion molecules belonging to an integrin family of macrophages by realtime RT-PCR. The liver was homogenized in ISOGEN to extract RNA according to the instruction manual. The concentration of RNA was measured using a spectrophotometer (ND-1000, NanoDrop Technologies, Inc.). Reverse transcription of mRNA from total RNA was performed using High Capacity cDNA Reverse Transcription Kit (Applied Biosystems) to synthesize cDNA. Gene expression analysis was performed by an intercalator method using SYBR green I or a method using Taqman Probe. Realtime RT-PCR was performed using 7900HT Fast Real-Time PCR System (Applied Biosystems). β-actin was used as an internal standard. After correction with β-actin, the expression levels of respective genes were compared with one another. The sequences of the used primers and probes are as follows:
β-actin :
   forward (5'-AGCCATGTACGTAGCCATCCA-3'),
   reverse (5'-TCACCGGAGTCCATCACAATG-3'),
   probe (VIC5'-TGTCCCTGTATGCCTCTGGTCGTACCAC-3'TAMRA),
CD11c:
   forward (5'-CTGGATAGCCTTTCTTCTGCTG-3'),
   reverse (5'-GCACACTGTGTCCGAACTC-3'),
TNF-α:
   forward (5'-CATCTTCTCAAAATTCGAGTGACAA-3'),
   reverse (5'-TGGGAGTAGACAAGGTACAACCC-3').

The statistical work was performed using the Windows (registered trademark) version of the SAS Preclinical Package (SAS Institute Japan Inc.).

### 5) Results

The compound A (3 mg/kg) administered group had no significant effect on TC and TG in the plasma as shown in FIG. 1. However, the group exhibited a significant decrease in TC and TG in the liver (FIG. 2) and significant decreases in inflammatory cytokine TNF-α expression in the liver and macrophage CD11c (FIG. 3). In other words, even if the dose of the compound A of the present invention was in a range that did not affect the plasma lipid, the compound had a direct effect on the liver and reduced the levels of cholesterol and triglyceride in the liver. Further, it inhibited the development of fatty liver and hepatic inflammation, i.e., characteristic conditions of NASH. This showed that the compound A of the present invention was useful as a prophylactic/therapeutic agent for NASH.

### Example 2 Effect in murine NASH-hepatoma models

Murine NASH-hepatoma models (brand name: C57BL/6 N-NASH mice) developed in Stelic Institute & Co. (See Japanese Patent Application Laid-Open (JP-A) No. 2009-178143) are provided by Charles River Laboratories Japan, Inc. The murine models develop NASH by initiation of insulin resistance and feeding with a high fat diet. The murine models show changes which histologically reflect the conditions of human NASH. The fatty liver (before 6 weeks old) progresses to NASH (7 weeks old), liver cirrhosis (after 10 weeks old), and liver cancer (after 16 weeks old). Accordingly, it is said that the models are useful in research and development of therapeutic drugs for NASH (See Data of STAM Mice C57BL/6N-NASH Model Mice (http://www.crj.co.jp/topics/pdf/69/6375030/B6N_NASH_leafle t_091019.pdf)).

Then, the effect of the compound A was examined using murine NASH-hepatoma models with a more serious condition than the knockout mice used in Example 1.

### 1) Used animal:

Male C57BL/6N-NASH mice (Charles River Laboratories Japan, Inc.) were used for the experiment. Streptozotocin was administered to the mice aged 1 to 2 days and they were fed with a high fat diet (D12492, Research Diet) from 4 weeks old. The mice were purchased at 6 weeks old and continuously fed with the same high fat diet (hereinafter, the mice are referred to as "C57BL/6N-NASH mice"). As a normal control group, normal mice (C57BL/6N, 6 weeks old, Charles River Laboratories Japan, Inc.) were purchased at the same time and they were fed with an ordinary diet (CE-2, CLEA Japan, Inc.) (hereinafter referred to as "normal mice").

### 2) Group constitution:

C57BL/6N-NASH mice were used for a control group and a compound A (100 mg/kg) administered group. The number of mice in each group was six. As normal mice (control group), 6 normal mice (C57BL/6N mice) were used.

### 3) Administration of drug:

The compound A and feeds were mixed to feed the mice. C57BL/6N-NASH mice with NASH aged 7 weeks were fed with the feed mixture. The dosing period was 5 weeks.

### 4) Observation and examination methods:

After the end of administration, the mice were fasted for 4 hours. The blood was collected from the abdominal portion of vena cava under anesthesia, and the plasma was fractionated. After collecting the blood, the liver was extracted. A part of the liver was frozen for preservation, and the other part was embedded in paraffin in a usual manner. Plasma lipid and plasma ALT levels were measured using an autoanalyzer
(Labospect003, Hitachi, Ltd.). Measurement of liver lipid was performed in the following manner. That is, chloroform and methanol (at a ratio of 2:1) were added to a part of the frozen and preserved liver, which was homogenized, and the resultant mixture was allowed to stand at 4°C overnight, followed by filtration. A part of the filtrate was fractionated, followed by evaporation and drying. The resultant product was dissolved in isopropanol. The concentrations of total cholesterol (TC) and triglyceride (TG) were measured using Cholesterol E-Test Wako and Triglyceride E-Test Wako, respectively. Further, paraffin sections and HE-stained specimens were produced to observe liver tissues.

### 5) Results

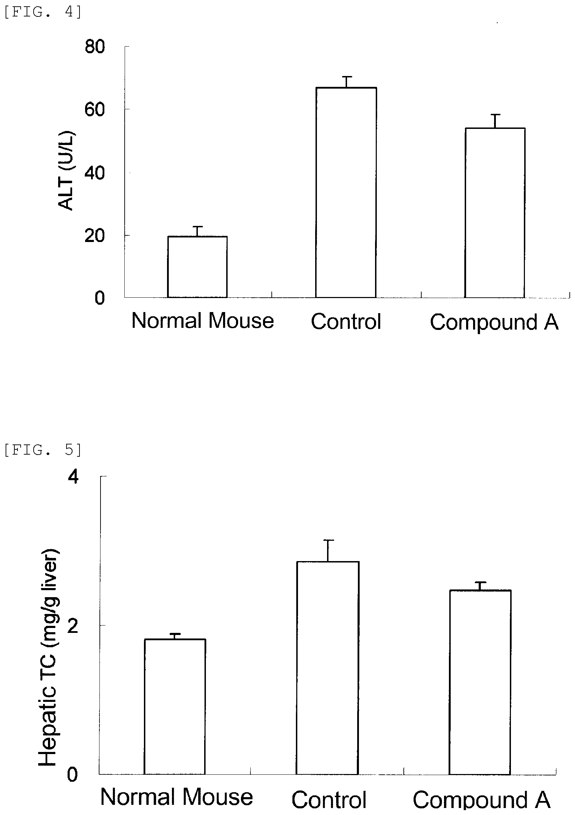

These results are shown in FIGS. 4 to 7.

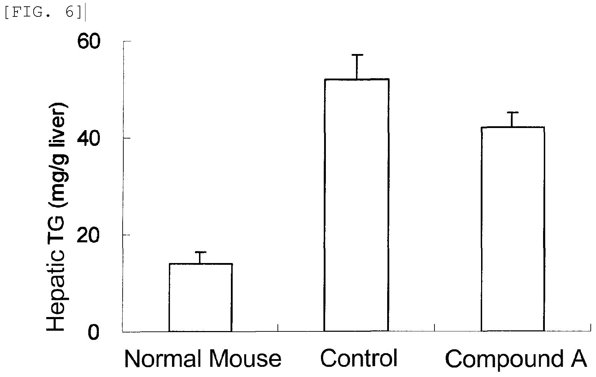

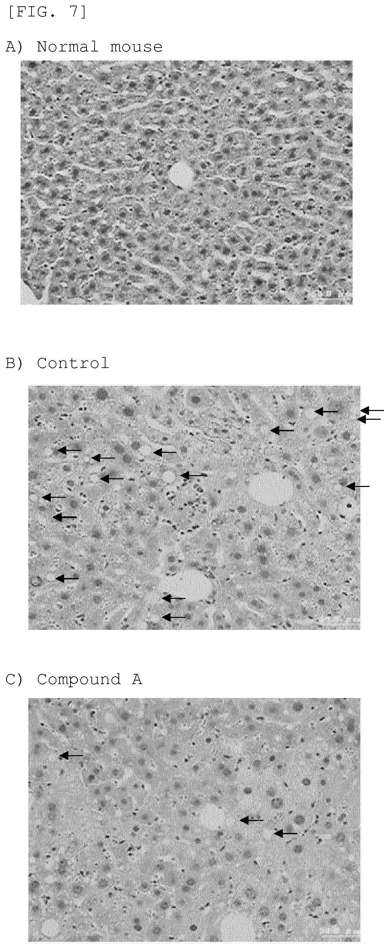

The compound A had no effect on the plasma lipid. The plasma ALT level as an indicator of liver damage in the murine NASH-hepatoma model control group was high as compared with that in the normal mouse group. However, the plasma ALT level in the compound A (100 mg/kg) administered group was reduced (FIG. 4). The hepatic TC and TG levels in the murine NASH-hepatoma model control group were high as compared with that in the normal mouse group. However, the plasma ALT level in the compound A administered group was reduced (FIGS.5 and 6). Further, the compound A inhibited the development of lipid-droplet
deposition, i.e., characteristic conditions of NASH (indicated by arrows in FIG. 7), which was observed in a control group of murine NASH-hepatoma models (FIG. 7). In other words, even if the administration of the compound A of the present invention was started after NASH development, the compound A in a range that did not affect the plasma lipid had a direct effect on the liver and reduced the levels of cholesterol and triglyceride in the liver. Further, it was not only reduced the plasma ALT level as an indicator of liver damage, but also inhibited the development of fatty liver, i.e., characteristic conditions of NASH. This showed that the compound A of the present invention was useful as a therapeutic agent for NASH.

### Example 3 Effect in KK-A^{y} mice fed with MCD diet

Rodent models fed with methionine-choline deficient diet (MCD diet) are widely used as NASH models (See Hebbard L, George J.:Animal models of nonalcoholic fatty liver disease Nat Rev Gastroenterol Hepatol. 2011 Jan; 8 (1) :35-44. Epub 2010 Nov 30.). Among them, models of KK-A^{y} mice are known to cause marked steatohepatitis and fibrosis (See Okumura K., Ikejima K., Kon K. , Abe W. , Yamashina S., Enomoto N. , Takei Y. and Sato N. (2006) Exacerbation of dietary steatohepatitis and fibrosis in obese, diabetic KK-Ay mice Hepatology Research 2006 36:3 (217-228)). It is considered that the mice are excellent models to examine the drug effect on NASH.

Then, an effect of the compound A on the present model was examined.

### 1) Used animal:

Male KK-A^{y} mice (KK-A^{y}/TaJcl mice, 12 weeks old, were purchased from CLEA Japan, Inc.) were used for the experiment. The mice were fed with the MCD diet for 7 weeks to cause NASH.

### 2) Group constitution:

The mice were separated into a control group, a compound A (30 mg/kg) administered group, and a compound A (200 mg/kg) administered group. The drug and feeds were mixed to feed the mice. The dosing period was 7 weeks.

### 3) Administration of drug:

The compound A and feeds were mixed to feed the mice. The mice was fed with the MCD diet, and fed with the feed mixture at the same time.

### 4) Observation and examination methods:

The methods were performed, in the same manner as in the case of the murine NASH-hepatoma models described in Example 2. In addition to the HE-stained specimens for observing liver tissues, Azan-stained specimens were also prepared.

### 5) Results

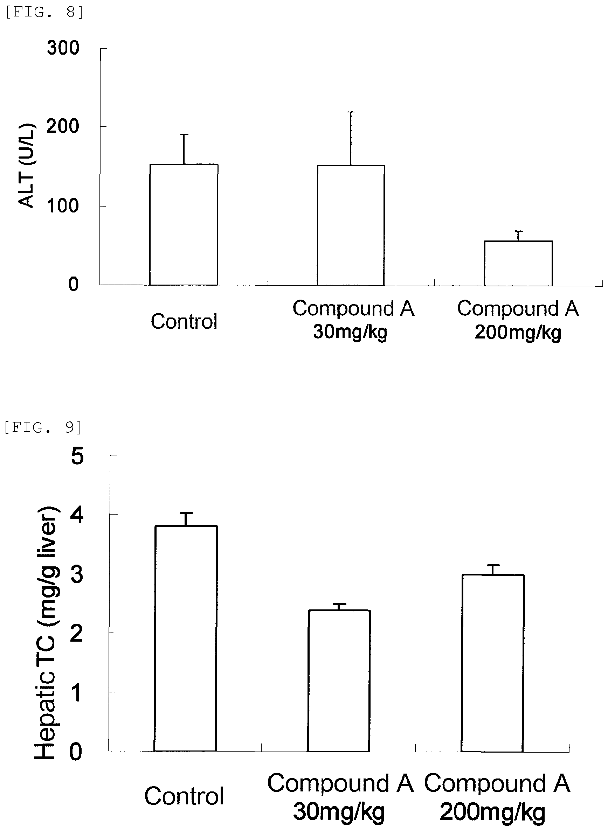

These results are shown in FIGS. 8 to 12.

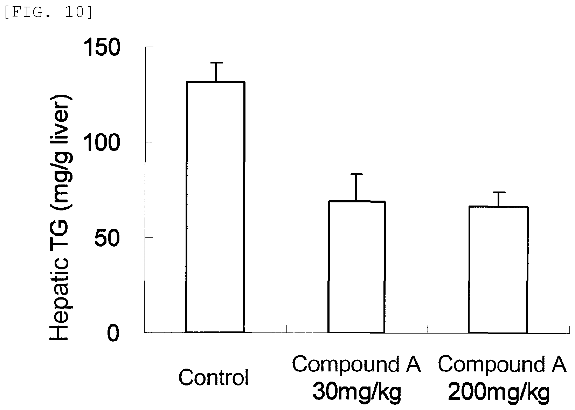

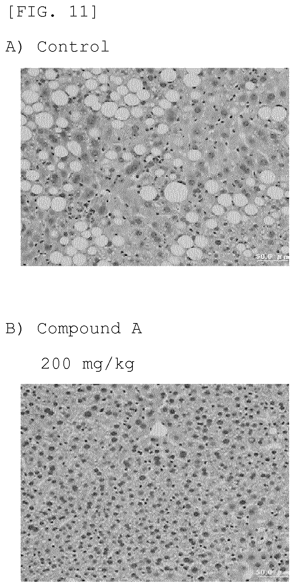

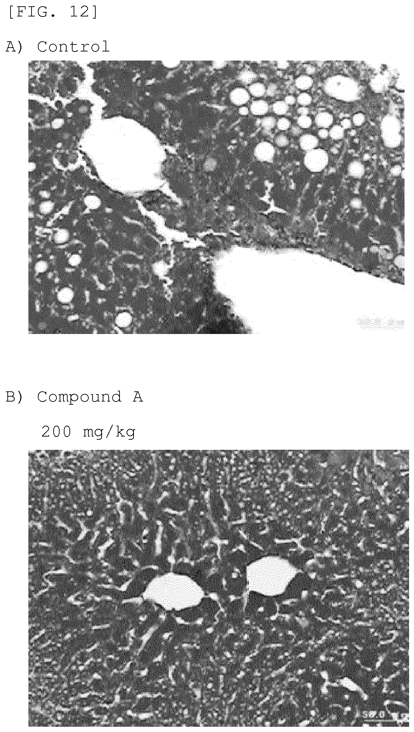

The compound A had no effect on the plasma lipid. The compound A (200 mg/kg) significantly reduced the plasma ALT level as an indicator of liver damage (See FIG. 8). The compound A significantly reduced the hepatic TC and TG levels (See FIGS. 9 and 10). Further, the compound A inhibited the development of lipid-droplet deposition and fibrosis in the liver, i.e., characteristic conditions of NASH (See FIGS. 11 and 12). In other words, even if the dose of the compound A of the present invention was in a range that did not affect the plasma lipid, the compound had a direct effect on the liver and reduced cholesterol and triglyceride levels in the liver. Further, it was not only reduced the plasma ALT level as an indicator of liver damage but also inhibited the development of fatty liver and fibrosis in the liver, i.e., characteristic conditions of NASH. This showed that the compound A of the present invention was useful as a prophylactic/therapeutic agent for NASH.

### INDUSTRIAL APPLICABILITY

In the present invention, it has been found out that 2-[4-[2-(benzimidazol-2-ylthio)ethyl]piperazin-1-yl]-N-[2,4 -bis(methylthio)-6-methyl-3-pyridyl]acetamide, an acid-addition salt thereof, or a solvate of the same has a hepatic selective total cholesterol-reducing effect, a triglyceride-reducing effect, an inhibiting effect for inflammatory cytokine expression in the liver, and an inflammatory macrophage-reducing effect for the first time. There is provided a low-molecular weight prophylactic and/or therapeutic agent for non-alcoholic steatohepatitis. Since the present invention can be used as a crude material, it is useful in pharmaceutical industries and has industrial applicability.

## Claims

1. 2-[4-[2-(benzimidazol-2-ylthio)ethyl]piperazin-1-yl]-N-[2,4-bis(methylthio)-6-methyl-3-pyridyl]acetamide, an acid-addition salt thereof, or a solvate of the same for use in the treatment and/or prophylaxis of non-alcoholic steatohepatitis.

2. A compound for use according to claim 1, wherein
2-[4-[2-(benzimidazol-2-ylthio)ethyl]piperazin-1-yl]-N-[2,4-bis(methylthio)-6-methyl-3-pyridyl]acetamide, an acid-addition salt thereof, or a solvate of the same is monohydrochloride of
2-[4-[2-(benzimidazol-2-ylthio)ethyl]piperazin-1-yl]-N-[2,4-bis(methylthio)-6-methyl-3-pyridyl]acetamide.

## Patentansprüche

1. 2-[4-[2-(Benzimidazol-2-ylthio)ethyl]piperazin-1-yl]-N-[2,4-bis(methylthio)-6-methyl-3-pyridyl]acetamid, ein Säureadditionssalz davon oder ein Solvat davon zur Verwendung zur Behandlung und/oder Prophylaxe von nicht-alkoholischer Steatohepatitis.

2. Verbindung zur Verwendung nach Anspruch 1, wobei 2-[4-[2-(Benzimidazol-2-ylthio)ethyl]piperazin-1-yl]-N-[2,4-bis(methylthio)-6-methyl-3-pyridyl]acetamid, ein Säureadditionssalz davon oder ein Solvat davon das Monohydrochlorid von 2-[4-[2-(Benzimidazol-2-ylthio)ethyl]piperazin-1-yl]-N-[2,4-bis(methylthio)-6-methyl-3-pyridyl]acetamid ist.

## Revendications

1. 2-[4-[2-(Benzimidazol-2-ylthio)éthyl]pipérazin-1-yl]-N-[2,4-bis(méthylthio)-6-méthyl-3-pyridyl]acétamide, un sel d'addition d'acide de celui-ci ou un solvate de celui-ci pour l'utilisation dans le traitement et/ou la prophylaxie de la stéatohepatite non alcoolique.

2. Composé pour l'utilisation selon la revendication 1, où le 2-[4-[2-(Benzimidazol-2-ylthio)éthyl]pipérazin-1-yl]-N-[2,4-bis(méthylthio)-6-méthyl-3-pyridyl]acétamide, un sel. d'addition d'acide de celui-ci ou un solvate de celui-ci est le monohydrochlorure de 2-[4-[2-(Benzimidazol-2-ylthio)-éthyl]-pipérazin-l-yl]-N-[2,4-bis(méthylthio)-6-méthyl-3-pyridyl]-acétamide.
